(19) **Europäisches Patentamt European Patent Office Office européen des brevets**

(11) **EP 1 432 738 B1**

(12) **EUROPEAN PATENT SPECIFICATION**

(45) Date of publication and mention
of the grant of the patent:
**04.03.2009 Bulletin 2009/10**

(51) Int Cl.:
*C07K 19/00* (2006.01)    *A61K 47/38* (2006.01)
*C07K 16/16* (2006.01)

(21) Application number: **02800565.0**

(22) Date of filing: **19.09.2002**

(86) International application number:
**PCT/EP2002/010523**

(87) International publication number:
**WO 2003/031477 (17.04.2003 Gazette 2003/16)**

(54) **CARBOHYDRATE BINDING DOMAIN CONTAINING FUSION PROTEINS FOR DELIVERY OF THERAPEUTIC AND OTHER AGENTS, AND COMPOSITIONS CONTAINING THEM**

KOHLENHYDRATBINDUNGSDOMÄNE ENTHALTENDE FUSIONSPROTEINE ZUR VERABREICHUNG VON THERAPEUTISCHEN UND ANDEREN STOFFEN UND ZUSAMMENSETZUNGEN IN DENEN DIE FUSIONSPROTEINE ENTHALTEN SIND

PROTEINES DE FUSION CONTENANT UN DOMAINE DE LIAISON AUX HYDRATES DE CARBONE POUR LA LIBERATION D'AGENTS THERAPEUTIQUES ET AUTRES AGENTS, ET COMPOSITIONS LES RENFERMANT

(84) Designated Contracting States:
**AT BE BG CH CY CZ DE DK EE ES FI FR GB GR IE IT LI LU MC NL PT SE SK TR**

(30) Priority: **03.10.2001 EP 01308467**

(43) Date of publication of application:
**30.06.2004 Bulletin 2004/27**

(73) Proprietors:
• **Unilever N.V.**
**3013 AL Rotterdam (NL)**
Designated Contracting States:
**AT BE BG CH CZ DE DK EE ES FI FR GR IT LI LU MC NL PT SE SK TR**
• **Unilever PLC**
**London**
**EC4Y 0DY (GB)**
Designated Contracting States:
**CY GB IE**

(72) Inventors:
• **BARNWELL, Stephen George**
**Wirral, Merseyside CH63 3JW (GB)**
• **PARRY, Neil James**
**Bedfordshire MK44 1LQ (GB)**

(74) Representative: **Joppe, Hermina Laura Petronella et al**
**Unilever Patent Group**
**Olivier van Noortlaan 120**
**3133 AT Vlaardingen (NL)**

(56) References cited:
WO-A-01/46356          WO-A-01/46357
WO-A-99/36469          WO-A-99/57154
WO-A-99/57155          US-A- 5 137 819
US-A- 5 837 814

• LINDER M ET AL: "CHARACTERIZATION OF A DOUBLE CELLULOSE-BINDING DOMAIN" JOURNAL OF BIOLOGICAL CHEMISTRY, AMERICAN SOCIETY OF BIOLOGICAL CHEMISTS, BALTIMORE, MD, US, vol. 271, no. 35, 30 August 1996 (1996-08-30), pages 21268-21272, XP002059599 ISSN: 0021-9258

**Description**

**TECHNICAL FIELD**

The present invention generally relates to fusion proteins which are useful for target-delivering agents of choice to specific sites of a living organism or other predominantly therapeutically useful surfaces including materials comprising medical devices. The invention further relates to compositions comprising such fusion proteins which are useful in a variety of applications, in particular the medical field, in the agrochemical field and in the field of paper processing.

**BACKGROUND AND PRIOR ART**

Although the effect of a particular pathology often is manifest throughout the body of the afflicted person, generally, the underlying pathology may affect only a single organ or tissue. In many cases, drugs are the treatment of choice for a patient suffering a particular disease. It is rare, however, that a drug will target only the diseased tissue or organ or be released at the correct rate to be most effective. More commonly, drug treatment results in undesirable side effects due, for example, to generalized toxic effects throughout the patient's body.

The undesirable side effects that can occur when drugs are used to treat a disease most often are due to the inability of the drug to specifically target the diseased organ or tissue or their release at the incorrect rate from the dosage vehicle, either too quickly leading to unwanted side effects, too slowly so as not to control symptoms, or at an inappropriate location.

For example, a cancer chemotherapeutic agent that targets rapidly proliferating cells would be useful to kill rapidly dividing cancer cells. However, such an agent also kills normal proliferating hematopoietic and epithelial cells. Thus, the dose of such a drug that can be administered to a patient is limited due to its toxic effect on normal cells.

Efforts have been made to increase the target specificity of various drugs and modify their release characteristics e.g., rapid release, delayed release, sustained release or a combination thereof. In some cases, a particular cell type present in a diseased tissue or organ may express a unique cell surface marker. In such a case, an antibody can be raised against the unique cell surface marker and a drug can be linked to the antibody. Upon administration of the drug/antibody complex to the patient, the binding of the antibody to the cell surface marker results in the delivery of a relatively high concentration of the drug to the diseased tissue or organ. Similar methods can be used where a particular cell type in the diseased organ expresses a unique cell surface receptor or a ligand for a particular receptor. In these cases, the drug can be linked to the specific ligand or to the receptor, respectively, thus providing a means to deliver a relatively high concentration of the drug to the diseased organ.

Cellulose binding domains ("CBDs") have been described as useful agents for the attachment of molecular species to cellulose, and have shown to be effective purification tools (US 5738984, US 6124117). WO 94/24158 discloses a nucleic acid sequence encoding a CBD as well as a fusion protein comprising the CBD and a second protein, and the potential use of such molecules, including drug delivery, affinity separations, and diagnostic techniques. However, such steps disclosed when coupled to drugs involve the chemical conjugation of the drug to the CBD. This represents an additional cost implication and may result in unfavorable economics in terms of ratios of CBD molecules to drug molecules (at least one CBD per drug molecule would be required). Furthermore, the chemical coupling process itself may negatively affect the drug, and the number of drug molecules that can be targeted is limited.

Targeted delivery using cellulose binding domains has been previously described in an unrelated field of the art. WO 01/46357 discloses detergent compositions which are capable of delivering a so-called Benefit Agent to a fabric during a washing or rinsing process. Such compositions comprise a fusion protein comprising a cellulose binding domain and a domain having a high binding affinity for another ligand. The fusion protein is bi-functional in its binding ability, whereby the cellulose binding domain region binds to cellulosic based materials and the second domain binds to another ligand.

WO 98/00500 discloses detergent compositions wherein a Benefit Agent is delivered onto fabric during the wash cycle by means of a peptide or protein Deposition Aid having high affinity for fabric, for example the cellulose binding domain of a cellulase enzyme, to which it is attached or absorbed. The Benefit Agent is a fabric softening agent, perfume, photoprotective agent, dye fixative agent, and the like.

Antibodies have also been shown to be effective in targeting liposomes to tumor cells (Bendas G. Biodrugs 15 (4):215-224 (2001)), but this relies on the incorporation of anti-bodies into the outer surface/membrane of the particles known also as immuno-liposomes.

There remains a need for systems that can deliver a therapeutic benefit agent (e.g., for pharmaceutical or agrochemical purposes) in a relatively high amount compared to the amount of antibody that is used.

There is also a need for systems that do not require covalent bonding of the antibody to the benefit agent.

Surprisingly, it has now been found that fusion proteins containing the cellulose binding domain or alternatively a binding domain recognising a carbohydrate or polysaccharide other than cellulose can be further adapted for use in pharmaceutical and other applications.

## SUMMARY OF THE INVENTION

The present invention provides a method of delivering an agrochemical benefit agent to a site in or on a plant, which comprises treating the plant with an agrochemical composition or kit of parts comprising (1) a micro-particle or micro-capsule comprising at least one pharmaceutical or agrochemical benefit agent and (2) a fusion protein which comprises a first binding domain which is a carbohydrate binding domain and a second binding domain capable of binding to (a) a ligand or specific site which forms part of a plant, or (b) said microcapsule or microparticle, wherein the first or second binding domain is capable of binding to said microcapsule or microparticle.

Preferably, the carbohydrate binding domain is capable of recognizing a useful binding site or a microcapsule or micro-particle containing at least one benefit agent. A preferred carbohydrate binding domain is a cellulose binding domain. Preferably, the second binding domain is an antibody or fragment thereof. More preferably, the antibody or fragment thereof is a Heavy Chain antibody selected from the group of Camelidae. The fusion protein may comprise one or more further optional binding domains. The further optional binding domain(s) of the fusion protein is a binding domain which is capable of binding to a therapeutic agent, a therapeutically useful binding site or a molecule or compound associated with it (for example, a molecule or substance put into the surface of a micro capsule or a molecule complexed or otherwise associated with said therapeutically useful binding site to enable the binding domain to recognise it) or, more preferably, a micro-particle or micro-capsule containing a high payload of at least one therapeutically useful agent. In some circumstances more than one binding domain will be able to recognise a disease site or otherwise therapeutically useful binding site.

Preferred polysaccharide binding domains include cellulose binding domains obtainable from a fungal enzyme origin such as _Humicola, Trichoderma, Thermomonospora, Phanerochaete, Aspergillus_ or from a bacterial enzyme origin such as _Bacillus, Clostridium, Streptomyces, Cellulomonas_ and _Pseudomonas_. Suitable sources of cellulose binding domains are disclosed in US 6331416.

These and other aspects of the present invention will be explained hereinafter in some more detail.

## BRIEF DESCRIPTION OF THE DRAWINGS

Figure 1 shows the ability of a fusion protein to deposit a target molecule to a cellulosic material.

Figure 2 shows the improved delivery of a molecule to a substrate in the presence of a fusion protein.

Figure 3 shows the deposition of molecules to tea bag paper in the presence of the fusion protein.

Figure 4 shows the delivery of molecules to salivettes using a fusion protein.

Figure 5 shows the deposition of molecules to cotton buds.

Figure 6 shows the results of deposition and binding of latex nanoparticles using a fusion protein.

Figures 7 and 8 show the deposition and binding of 10 and 31$\mu$m latex beads, respectively.

Figures 9, 10 and 11 show the increased deposition of coacervate particles onto freshly cut grass using a CBD fusion protein.

Figure 12 is a schematic representation of the principle of the present invention showing as a preferred embodiment a self assembling targeted controlled release system using a polysaccharide binding domain-antibody fusion protein.

## DETAILED DESCRIPTION OF THE INVENTION

The systems used in the method of the present invention may be in the form of a composition e.g., a homogeneous or non-homogeneous mixture of the fusion protein and the micro-particles or microcapsules. The system may alternatively be in the form of a kit of parts which comprises the fusion protein packaged separately from the micro-particles or microcapsules; the fusion protein and the micro-particles or microcapsules may be mixed before use or used separately i.e., the fusion protein and the microparticles or microcapsules may be intended for simultaneous, separate or sequential use. The kit of parts preferably comprises a two part package, typically together with instructions for use.

By "benefit agent", as used herein, we mean any suitable agent which can be used for benefitting - for human welfare - an organism or tissue or surface to be treated or intended to be treated. Such benefit agents include, for example, therapeutic agents, in particular relatively small organic molecules, peptides, proteins, DNA, RNA, vaccines, vectors used in gene therapy, live or dead micro-organisms, or any other substance or system used to treat or prevent a disease state in plants or animals, including humans. Thus, when the composition or kit of parts is for pharmaceutical use, the benefit agent is preferably selected from therapeutic small molecules, peptides, proteins, nucleic acids and vaccines.

Therapeutic agents are for example, proteins, peptides, nucleic acids and small organic molecules, for example local anesthetics (such as cocaine, procaine and lidocaine), hypnotics and sedatives (such as barbiturates, benzodiazepines and chloral derivatives), psychiatric agents (such as phenothiazines, tricyclic antidepressants and monoamine oxidase inhibitors), anti-epilepsy compounds (such as hydantoins), L-dopa, opium-based alkaloids, analgesics, antiinflammatories, allopurinol, cancer chemotherapeutic agents, anticholinesterases, sympathomimetics (such as epinephrine, salbutamol and ephedrine), antimuscarinics (such as atropine), β-adrenergic blocking agents (such as phentolamine), β-adrenergic blocking agents (such as propranolol), ganglionic stimulating and blocking agents (such as nicotine), neuromuscular blocking agents, autacoids (such as antihistamines and 5-HT antagonists), prostaglandins, plasma kinins (such as bradykinin), cardiovascular drugs (such as digitalis), antiarrhythmic drugs, antihypertensives, vasodilators (such as amyl nitrate and nitroglycerin), diuretics, oxytocin, antibiotics, anthelminthics, fungicides, antiviral compounds (such as acyclovir), anti-trypanosomals, anticoagulants, sex hormones (for example for HRT or contraception), insulin, alprostidil, blood-clotting factors, calcitonin, growth hormones, vaccines, constructs for gene therapy and steroids. The recipient may be a human or any other vertebrate, preferably a mammal, bird or fish for example a cow, sheep, horse, pig, chicken, turkey, dog, cat or salmon, or a plant, especially for DNA transformation of the plant.

Conditions in which the benefit agent may be useful for treatment or prophylaxis or for the alleviation of symptoms include, for example, bacterial infections, viral infections, cancer, CNS disorders, cardiovascular disease and allergic diseases. Specific examples of conditions include diabetes, genetic disorders (treated by gene therapy), pain, addiction, insomnia and sleep disorders, eating disorders, epilepsy, psychiatric disorders, Parkinson's disease, Alzheimer's disease, depression, stroke, rheumatic diseases, asthma, eczema, HIV and AIDS, gastric disorders and hypertension.

In the agricultural composition or kit of parts of the invention the benefit agent is selected from pesticides (e.g., insecticides), herbicides, fungicides, plant growth stimulating agents, insect attractants or repellents (such as pheromones) and crop protecting agents.

Examples of agrochemicals suitable for use in the invention include glyphosate, fomesafen, glufosinate, mecoprop p, methylchlorophenoxy acetic acid, propamocarb, fosetyl, triasulfuron, tribenuron, metasulfuron, thifensulfuron, flupysulfuron, iodosulfuron, rimsulfuron, nicosulfuron, cinosulfuron, bensulfuron, trifloxysulfuron, flumetsulam, metosulam, chloransulam, floransulam, imazethabenz, imazethapyr, imazaquin, imazamox, flucarbazone, propoxycarbazine, amicarbazone, clodinafop, fenoxaprop, diclofop, propaquizafop, quizalofop, fluazifop, cyhalofop, haloxyfop, sethoxydim, clethodim, tralkoxydim, dicamba, clopyralid, 2,4-D, fluroxypyr, amicarbazone, azafenidin, benfluamid, benzfendizone, benzobicyclon, cinidon-ethyl, diclosulam, fentrazamid, flufenacet, flufenpyr, foramsulphuron, indanofan, mesosulphuron, oxaziclomefone, penoxsulam, pethoxamid, picolinafen, profoxidim, profluazol, propoxycarbazone, pyraflufen, pyrazogyl, sulphosulphuron, tepraloxydim, tritosulphuron and analogues and/or agrochemically acceptable salts thereof.

The carbohydrate binding domain of the fusion protein according to the present invention is firstly and preferably used to bind a particulate containing a high payload of at least one agrochemical benefit agent whereas the other high affinity binding site would optionally bind a micro-capsule or micro-particle containing at least one benefit agent, in particular a therapeutic agent or a disease site or otherwise therapeutically useful binding site. Alternatively, the present invention includes an additional binding site that optionally binds another micro-capsule or micro-particle containing at least one therapeutic agent (in addition or instead), or a disease site or otherwise therapeutically useful binding site.

The fusion protein may further comprise one or more additional binding domains i.e., in addition to the first and second binding domains. For example, the fusion protein may comprise a third binding domain which is capable of binding to said microparticle or micro-capsule and/or a binding domain which is capable of binding to a therapeutic agent or a molecule or compound associated with it. Also, the fusion protein may comprise two or more of the first binding domains and/or two or more of the second binding domains.

It will be understood that there is not intended to be any significance attached herein to the words first, second, third and related terms in connection with the definition of the binding domains, other than for the purposes of identifying different parts or regions of the fusion protein. Thus, the first and second binding domains can be present in the fusion protein in any order and the fusion protein may be produced from any genetic format (for example, the first binding domain need not necessarily be formed first nor be the major part of the molecule).

The advantage of this construct is that site-specific delivery is combined with, e.g., a micro-capsule containing a high payload of at least one agrochemical benefit agent. This is different from other attempts at high payload site specific delivery as they have been limited by either the number of therapeutic molecules that can be linked to a single antibody or require the targeting moiety (e.g. antibody) to be incorporated into the surface of the micro-capsule such as in the case of immuno-liposomes.

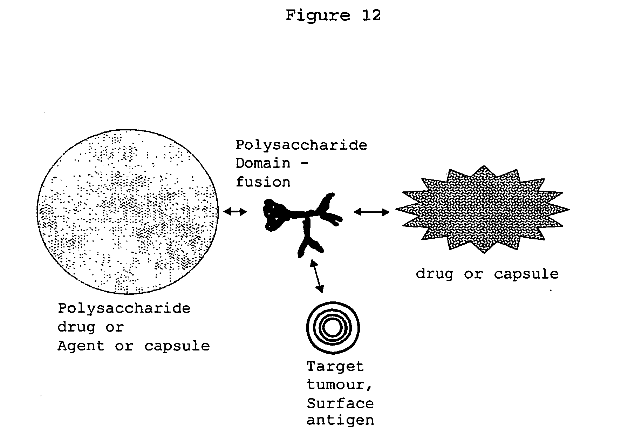

In another embodiment of the invention, the fusion proteins facilitate the biphasic delivery of agrochemical benefit agents from the same delivery vehicle. This aspect is represented diagrammatically in Figure 12. Biphasic delivery of therapeutic agents and pharmaceutical excipients has previously been shown to enhance the therapeutic effect of pharmaceutically useful compounds, such as propranolol in clinical trials (Barnwell SG et al, 1996, International Journal of Pharmaceutics, 128, 145-154). Biphasic delivery includes, for example, the release of the same therapeutic agent under two separate conditions, for example rapid and sustained release or alternatively the release of two or more therapeutic agents at different rates or under different external conditions, for example both rapid release or both sustained release or one rapid release and one sustained release or pH dependent release. Biphasic release may be achieved by using a fusion protein containing a carbohydrate binding domain bound to a micro-capsule comprising a carbohydrate recognised by the binding domain of the fusion protein and at least one therapeutic agent together with a micro-capsule together with an additional binding domain recognising and binding to a micro-capsule containing at least one therapeutic agent (e.g., an anti-body recognising the gelatine comprising a gelatine micro-capsule, or another moiety on a capsule surface). Release (e.g., rapid release, sustained release, pH dependent release) of the therapeutic agent(s) from each of the two bound micro-capsules is usually determined by the composition of the micro-capsules.

In a further embodiment of the present invention, a fusion protein is provided that combines the advantages of each of the delivery vehicles described above. In this instance, the fusion protein is furnished with at least two additional binding domains in addition to the carbohydrate binding domain. This type of construct enables the disease specific delivery of a biphasic release delivery vehicle containing at least one agrochemical benefit agent. Such fusion variants contain more than two protein domains specified such that the fusion contains at least one carbohydrate binding domain and at least one protein domain exhibiting a binding affinity to another molecule or surface.

In still another embodiment of this invention, the use of the carbohydrate-binding domain of the fusion protein as the targeting moiety is envisaged. In this case, the carbohydrate binding domain is applied to bind a surface containing a carbohydrate, e.g. plant leaves, stems or roots, whereas at least one other domain on the fusion protein is used to bind a micro-capsule containing an agent useful in agrochemical applications.

The present invention is advantageous over the prior art in that it is capable of greatly increasing the amounts of agrochemical benefit agent that can be delivered using a single fusion protein by the use of micro-capsules or micro-particulate constructs that comprise a high payload of at least one benefit agent.

It will be appreciated that in the compositions and kit of parts used in the method of the invention, the first or second binding domain is capable of binding to said microcapsule or microparticle in order that the microparticle can be delivered. Thus, either the first binding domain is capable of binding to the microcapsule or microparticle and the second binding domain is targeted to the intended site of delivery (e.g., an antibody directed to a particular cell surface antigen) or the first binding domain directs the delivery of the fusion protein (e.g., binding to a carbohydrate-containing substrate such as the surface of a plant) and the second binding domain is capable of binding to the microcapsule or microparticle.

In one embodiment, the invention involves the use of a fusion protein for agrochemical purposes. It will be appreciated that the fusion protein may be used with or without the microparticles and microcapsules. Thus, when used without the microparticles or microcapsules, the fusion protein may be used to deliver a single molecule to a carbohydrate-containing site or a plurality of molecules to a carbohydrate-containing site, depending on the nature of the second binding domain. For example, when the second binding domain is an antibody, the fusion protein may be used to deliver a single molecule to a carbohydrate-containing site. When the second binding domain can bind a plurality of molecules, more than one molecule may be delivered to a carbohydrate-binding site from the same fusion protein.

The fusion protein according to the invention and its various elements will now be described in some further detail.

The Carbohydrate Binding Domain

In one aspect, the present invention relates to a fusion protein comprising a carbohydrate binding domain and at least one other domain having a high binding affinity for another ligand. The latter preferably comprises a micro-capsule or construct comprising a high payload (i.e., preferably more than 10, more preferably more than 100, even more preferably more than 1,000, most preferably more than 10,000 molecules per micro-capsule, microparticle or construct) of at least one therapeutic agent or optionally a disease site or otherwise therapeutically useful binding site where the carbohydrate binding domain may be used either to bind a micro-particle containing a high payload of at least one therapeutically useful agent or optionally bind to the surface of a therapeutically useful surface such as a bandage, dressing or medical device.

Cellulose Binding Domains mentioned before are examples of polysaccharide binding domains (PBDs) which can also be referred to as Carbohydrate Binding Domains. The term carbohydrate binding domain also includes protein sequences such as Starch Binding Domains, Mannose Binding Domains, Xylan Binding Domains, and Chitin Binding Domains. Examples of the range of these polysaccharide binding domains available are described by Henrissat B.,

Davies G.J. (Plant Physiology (2000) 124(4):1515-1519) and in US6331416. The term Carbohydrate Binding Domain will be collectively used hereinafter for any of such binding domains which can be used as part of the fusion protein according to the present invention, unless indicated otherwise, and may also be referred to as CBD.

A preferred binding domain is the Cellulose Binding Domain which will be used hereinafter as a typical example of the carbohydrate binding domain part of the fusion protein described and claimed in the present invention. Thus, the abbreviation CBD is used hereinafter to indicate Carbohydrate Binding Domains in general, whereas a Cellulose Binding Domain is a typical and preferred example of the broader definition of Carbohydrate Binding Domains so that no confusion will arise.

A Cellulose Binding Domain is a polypeptide which has high affinity for or binds to water soluble or water insoluble forms of cellulose and chitin, including crystalline forms. Cellulose Binding Domains are found as integral parts of large protein complexes consisting of two or more different polypeptide domains, for example in hydrolytic enzymes (hydrolases) which typically are composed of a catalytic domain containing the active site for substrate hydrolysis, and a Carbohydrate Binding Domain such as a Cellulose Binding Domain for binding to the insoluble matrix. Such enzymes can comprise more than one catalytic domain and one, two or three CBDs and optionally one or more polypeptide regions linking the CBD(s) with the catalytic domain(s), the latter regions usually being denoted a "linker". Examples of hydrolytic enzymes comprising a CBD are cellulases, xylanases, mannanases, arabinofuranosidases, acetyl esterases and chitinases. CBDs have also been found in algae, e.g. the red alga *Porphyra purpurea* as a non-hydrolytic polysaccharide binding protein, see Peter Tomme et al., "Cellulose Binding Domains: Classification and Properties" in "Enzymatic Degradation of Insoluble Carbohydrates", John N. Saddler and Michael H. Penner (Eds.), ACS Symposium Series, No. 618, 1996. However, most of the known CBDs are from cellulases and xylanases.

In this context, the term "Cellulose Binding Domain" is intended to be understood as defined by Tomme et al., op. cit. This definition classifies more than 120 Cellulose Binding Domains into 10 families (I-X) which may have different functions or roles in connection with the mechanism of substrate binding. However, it is anticipated that new family representatives and additional CBD families will appear in the future.

A carbohydrate binding domain may be exemplified by a binding domain recognising cellulose. A cellulose binding domain is a part of many cellulolytic enzymes and can be obtained therefrom. Cellulose binding domains are also obtainable from xylanase and other hemicellulase degrading enzymes. Preferably, the cellulose binding domain is obtainable from a fungal enzyme origin such as *Humicola, Trichoderma, Thermomonospora, Phanerochaete, Aspergillus* or from a bacterial enzyme origin such as *Bacillus, Clostridium, Streptomyces, Cellulomonas* and *Pseudomonas*. Other polysacharide binding sites of interest include those that recognise the polysaccharide components of glycoproteins found within animal tissues.

The second and possibly further binding domains

In the fusion protein according to the invention, the carbohydrate binding domain is fused to a second and, optionally, a third binding domain having high binding affinities for other ligands, respectively. Preferably, the carbohydrate binding domain (exemplified by a cellulose binding domain) is connected to the domain having a high binding affinity for another ligand by means of a linker consisting of about 0-20, preferably about 2-15, more preferably of 2-5 amino acid residues.

The second, optional third and any other binding domains may be derived from any molecules or fragments of molecules which are capable of exhibiting selective binding affinity, preferably they are peptides or proteins. The second domain having a high binding affinity for another ligand usually is an antibody or antibody fragment, although the invention is not limited to antibodies and antibody fragments, and other molecules having selective binding affinity may be used instead. Especially preferred are heavy chain antibodies such as found in Camelidae.

The fusion protein according to the invention may comprise more than two recognition domains. It is for example possible to produce a CBD fusion protein with more than one antibody domain, in which the antibody domains may bind to the same or bind to different antigens. Conversely, it is also possible in the CBD antibody fusion format to produce a molecule with one antibody domain with more than one CBD, whereby the CBDs incorporated may be identical sequences or may be isolated from more than one source, or modified varieties thereof.

The fusion protein is comprised of two or more binding domains whereby at least one binding domain is a carbohydrate binding domain and at least one domain having a high binding affinity for another ligand or molecule, such as an antibody domain or a fragment derived thereof.

Generally speaking, the degree of binding of a molecule A to another molecule B can be generally expressed by the chemical equilibrium constant $K_d$ resulting from the following reaction:

$$[A] + [B] \Leftrightarrow [A \equiv B]$$

The chemical equilibrium constant $K_d$ is then given by:

$$K_d = \frac{[A]x[B]}{[A \equiv B]}$$

Whether the binding of a molecule to the target site/ligand is specific or not can be judged from the difference between the binding ($K_d$ value) of the molecule to one type of target site/ligand, versus the binding to another type of non-target site ligand material. For pharmaceutical and agrochemical applications, said ligand material will form part of or be associated with a therapeutic agent, preferably in a microcapsule or other construct. In this aspect of the invention, the CBD region of the fusion protein binds to a carbohydrate containing material and the high affinity domain region binds to the therapeutic agent construct. Alternatively, this approach can be reversed whereby the therapeutic site is targeted by the high affinity domain of the fusion protein and the carbohydrate binding domain region binds to a carbohydrate based or carbohydrate containing therapeutic agent construct or micro-capsule. However, it will usually be more convenient to measure $K_d$ values and differences in $K_d$ values on other materials such as a polystyrene microtitre plate or a specialised surface in an analytical biosensor. The difference between the two binding constants should be minimally 10, preferably more than 100, and more preferably, more than 1000. Typically, the reagent should bind to the target site/ligand, with a $K_d$ lower than $10^{-3}$ M, preferably lower than $10^{-6}$ M and could be $10^{-9}$ M or even less. Higher binding affinities ($K_d$ of less than $10^{-5}$ M) and/or a larger difference between the one type of target-site/ligand and another type of target site/ligand (or background) binding would increase the deposition of the benefit agent. In summary, improved delivery of therapeutic agents would in addition to potentially improving therapeutic efficacy would also offer the opportunity to lower the dose of the therapeutic agent while maintaining efficacy.

Several classes of agents or molecules can be envisaged which are capable of binding to a specific targeted site, for example in a diseased tissue or organ. As stated before, an important and preferred class of such agents or molecules is the class of antibodies which will be discussed below in more detail. Other classes, such as, e.g. peptides and peptidomimics, been mentioned and discussed in WO 01/46357 and WO 98/00500 the contents of which are herein incorporated by reference. The use of such agents or molecules is also contemplated in the present invention.

Antibodies

Antibodies are specific binding proteins. Their function in nature is to protect against disease by recognising (and binding) foreign bodies, such as viruses or Bacteria, but not self-cells. Furthermore, methods are well-known in the art to generate antibodies that are specific for almost any protein, organic molecule, or cell surface, that is likely to be encountered. This binding specificity has been exploited in the biotechnology industry, principally for medical diagnostics. For example, many home-based pregnancy test kits comprise an antibody that specifically binds to the pregnancy marker hormone, human chorionic gonadotropin (hCG), but not to other hormones present in urine.

More recently, the use of antibodies in laundry products has been described (Henkel, Procter and Gamble, Unilever). In particular, Unilever has described the use of stain-specific antibodies to target bleaching enzymes exclusively to stains but not to dyes - thus achieving efficient stain removal without damaging surrounding fabric.

Antibodies are well known examples of molecules which are capable of binding specifically to compounds against which they were raised. Antibodies can be derived from several sources. From mice, monoclonal antibodies can be obtained which possess very high binding affinities. From such antibodies, Fab, Fv or scFv fragments, can be prepared which have retained their binding properties. Such antibodies or fragments can be produced through recombinant DNA technology by microbial fermentation. Well known production hosts for antibodies and their fragments are yeast, moulds or bacteria.

A class of antibodies of particular interest is formed by the Heavy Chain antibodies as found in Camelidae, such as the camel or the llama. The binding domains of these antibodies consist of a single polypeptide fragment, namely the variable region of the heavy chain polypeptide (HC-V). In contrast, in the classic antibodies (murine, human, etc.), the binding domain consists of two polypeptide chains (the variable regions of the heavy chain ($V_h$) and the light chain ($V_1$)). Procedures to obtain heavy chain immunoglobulins from Camelidae, or (functionalized) fragments thereof, have been described in WO-A-94/04678, and WO-A-94/25591, and are incorporated herein by reference. Alternatively, binding domains can be obtained from the $V_h$ fragments of classical antibodies by a procedure termed "camelization". Hereby the classical $V_h$ fragment is transformed, by substitution of a number of amino acids, into a HC-V-like fragment, whereby its binding properties are retained. This procedure has been described by Riechmann et al. in a number of publications (J. Mol. Biol. (1996) 259, 957-969; Protein. Eng. (1996) 9, 531-537, Bio/Technology (1995) 13, 475-479). Also HC-V fragments can be produced through recombinant DNA technology in a number of microbial hosts (bacterial, yeast, mould), as described in WO-A-94/29457.

Methods for producing fusion proteins that comprise an enzyme and an antibody or that comprise an enzyme and an antibody fragment are already known in the art. One approach is described by Neuberger and Rabbits (EP-A-0 194 276). A method for producing a fusion protein comprising an enzyme and an antibody fragment that was derived from an antibody originating in *Camelidae* is described in WO-A-94/25591. A method for producing bispecific antibody fragments is described by Holliger et al. (1993) PNAS 90, 6444-6448.

A particularly attractive feature of antibody binding behaviour is their reported ability to bind to a "family" of structurally related molecules. For example, in Gani et al. (J. Steroid Biochem. Molec. Biol. 1994 48, 277-282) an antibody is described that was raised against progesterone but also binds to the structurally-related steroids, pregnanedione, pregnanolone and 6-hydroxy-progesterone.

## Compositions

In the compositions, the fusion protein and the microparticles or the microcapsules are formulated together. In the kit of parts, the fusion protein and the microparticles or the microcapsules are formulated separately (i.e, as separate formulations).

Compositions and formulations of the invention may comprise a suitable carrier. Preferred compositions and formulations are pharmaceutical compositions where the carrier is a pharmaceutically acceptable carrier either in solid or in liquid form. Compositions and formulations are contemplated for agrochemical use where the fusion protein of the invention carries a high payload of a benefit agent of choice, such as a growth-stimulating or crop-protecting agent or a herbicide or pesticide, and the carrier is a suitable carrier for such use, e.g. on crops, either in solid or, preferably, in liquid form, e.g. water or another suitable solvent. Again, a skilled person will have no difficulty in selecting an appropriate carrier depending on the specific use of the fusion protein according to the invention. If desired, the active ingredient of the compositions and formulations of the invention can be brought in a form which facilitates its processing or uptake by the plant e.g. a suitable salt or ester.

Agrochemical compositions of the invention are compositions that are intended for application to growing plants, or areas where plants (especially food crops) are grown, in order to benefit the health and/or growth of the plant, either directly (for example, by acting on the plant) or indirectly (for example, by the control of insects). Agrochemical compositions may be in the form of solids (e.g., powders), semi-solids (e.g., gels or pastes) or liquids (e.g., emulsions, suspensions, dispersions or solutions). The compositions are preferably in the form of sprayable liquids, or liquid concentrates which are capable of being sprayed when mixed with water. The compositions preferably comprise a wetting agent, such as a surfactant, and may also comprise one or more other emulsifying agents.

## Fusion Proteins

The fusion proteins and compositions according to the present invention are prepared by methods which are well known in the art. The fusion proteins can be prepared, for example, starting from the disclosures of WO 01/46357 and/or WO 98/00500 and a skilled person will have no difficulty in making any modification or addition thereof based on the teaching of the present specification and his knowledge of the art. The same applies to the preparation of the compositions of the present invention.

## Micro-particles and Micro-capsules

The microparticles and microcapsules for use in the present invention may be produced by methods known in the art.

Microcapsules and microparticles useful in the invention preferably consist of populations in which at least 90% (more preferably at least 95%) of the particles have an average particle size (based on the maximum dimension of the particles, such as the diameter when they are spherical) of from 0.05 $\mu$m to 2.0 mm, more preferably from 0.5 $\mu$m to 200 $\mu$m, most preferably from 10 $\mu$m to 80 $\mu$m. Particle sizes can be determined by microscopy using standard graticule slides, as is well known in the art.

Micro-encapsulation is a generic term used to describe a variety of micro-particulate constructs that include microcapsules, micro-spheres, nano-particles, nano-capsules and liposomes. These constructs may be spherical, oblong, irregularly shaped, monolithic or aggregates comprising single or multiple walls. Micro-capsules may range in size from one micron to about 7mm with smaller particles often being termed nano-capsules or nano-spheres. Within this diverse group of micro-encapsulate descriptors two sub-categories of particle constructs are readily differentiated:

those with a discrete continuous outer coat, film or membrane where the core is mainly concentrated near the centre and termed reservoir design; and
those in which the core is uniformly dispersed throughout the matrix and called matrix design.

In pharmaceutical and biomedical applications these constructs are frequently termed either micro-capsules or micro-particles, respectively; the same terminology is used herein.

A variety of terms are used to describe the contents of micro-capsules including core material, core, substrate, ingredient, and active agent whereas the coating material is termed shell, coat, wall, encapsulating matrix, encapsulating agent and carrier.

A number of different techniques can be used to form micro-capsules or microparticles for use in the present invention. In general, methods of producing micro-capsules fall into three distinct categories:

Physico-chemical processes, including simple or complex coacervation (aqueous phase separation), emulsion solvent evaporation (organic phase separation), emulsion-solidification and liposome entrapment.

Chemical processes, including interfacial polymerisation and molecular inclusion.

Physical processes such as spray-drying, spray-coating, spray-granulation, prilling and extrusion.

The coating or shell material used for micro-encapsulation purposes include many natural and synthetic polymers typically forming between 1% and 70% of the micro-capsules by weight. Coating materials useful in the production of micro-capsules include: gums, for example gum arabic, agar, sodium alginate and carrageenan; carbohydrates, for example starch, dextran, sucrose and corn syrup; celluloses, for example carboxymethylcellulose, methylcellulose, ethylcellulose, nitrocellulose, acetylcellulose, cellulose acetate-phthalate and cellulose acetate-butylate-phthalate; lipids, for example wax, paraffin, tristearin, stearic acid, monoglycerides, diglycerides, beeswax, oils, fats and hardened oils; inorganics, for example calcium sulphate, silicates and clays; and proteins, for example, gluten, casein, gelatine and albumin.

Coating materials are selected on the basis of the chemical and physical chemical properties of the core material, the micro-encapsulation process to be used and the desired properties of the micro-capsules such as release characteristics. For example, water-soluble polymers are used to micro-encapsulate poorly soluble core materials, whereas water insoluble polymers are used with water soluble core materials. The overall performance of the coating polymers can be modified by altering coat thickness and the use of plastisizers, cross-linkers or multiple coatings, however in general the coating material should not either solubilise or react with the core material.

Examples of other polymers that may be used include those used in the preparation of bio-degradable microspheres such as poly(amides), poly(amino acids), poly(alkyl-$\alpha$-cyano acrylates), poly (esters), poly(orthoesters), poly (urethanes) and poly(acrylamides). Most frequently studied however are the thermoplastic poly(esters) polylactide (PLA), polyglycolide (PGA) and in particular poly(lactide-co-glycolide) (PLGA).

The physical micro-encapsulation process of spray-drying is the most commonly used technique of micro-encapsulation. Spray-drying is defined as the transformation of a fluid state starting material (solution, dispersion or paste) into a dried particulate form via the process of spraying into a hot drying medium. The micro-capsules formed by spray-drying fall within the matrix type design category with the contents existing as micro-particles or micro-droplets distributed within a dry solid support matrix. Although often considered a dehydration process micro-capsules are formed when active materials become trapped within the matrix formed from a polymer or melt. Despite a size distribution determined by many process variables particles formed using the spray-drying process are typically under $100 \mu m$ in size.

Utility

Compositions may be used in the pharmaceutical or healthcare fields. The compositions can be used to deliver a molecule or substance that is either biologically active (e.g., a pharmaceutical or an antibacterial or antifungal agent) or otherwise therapeutically useful for the treatment or prevention of disease or the alleviation or prevention of pain or discomfort. Specific examples of applications of the compositions are their use as delivery vehicles (e.g., to deliver biologically active molecules or substances to a desired site). Other medical applications include the manufacture or treatment of sterile articles for use in medicine such as bandages, dressings, medical devices and fabrics used in medicine. Examples include wound dressings (e.g., bandages, plasters and burn dressings), cotton buds, supports (e.g., for joints such as the knee, elbow, wrist or neck), cotton wool, wipes and tissues, and other specialist dressings. The fusion proteins may be used to deliver benefit agents such as antibacterial or antifungal agents to these articles. Other examples of medical applications include use in spray on skin, self-disinfecting surgical garments (including clothes, drapes and masks), swabs, dental articles and equipment (including toothbrushes, toothpicks and dental floss), surgical tape, brushes and combs, garments including socks for athletes foot or verucas, gloves or other garments for the treatment of eczema, hair accessories and hats, skin/nail and hair treatments, capsules, prosthetics and medical implants (e.g., fibreglass as used in casts for broken limbs), liners to fit between prosthetic limbs and their attachment point, chewing gum, toothpaste, medicated shampoos, and targeted sunscreen for garments (skin protection).

The fusion proteins have the advantage that they can be recharged with the benefit agent. Thus, a single treatment with the fusion protein can be followed by multiple treatments with the benefit agent which then has increased affinity for the article as a result of binding via the fusion protein that can remain bound to the surface of the article.

The compositions of the invention are used in the agrochemicals field. The compositions can be used to deliver materials that are beneficial for plants either directly, for example as nutrients or selective weedkillers, by killing or repelling plant pests or other pathogens (such as fungi) or indirectly by attracting beneficial insects or animals. Specific examples of applications include: the treatment of plant cell walls; killing or repulsion of plant pests; the attraction of beneficial animals and insects; the treatment of bark, labels, fence posts, decking, tree protectors or tape; the production of a nutrient-releasing, fungicidal or insecticidal article for insertion into the ground (e.g., a peg); targeting roots for destruction (i.e., getting rid of remaining tree roots); destruction of infestations; slow or delayed release of molecules for insecticidal action; mole deterent e.g., by release of the Imperial lily odour; cat deterrent; targeting nutrients to seeds; and bird repellent.

Compositions described herein can be used in the paper treatment, manufacture or processing e.g., on a large scale (greater than 100 kg of paper treated, manufactured or processed per day) in the paper industry. The invention therefore contemplates a method of treating paper that comprises applying to the paper a fusion protein which comprises a first binding domain which is a carbohydrate binding domain and a second binding domain capable of binding to (a) a microcapsule or micro-particle, wherein the microcapsule or microparticle comprises a material that is beneficial in the processing of paper or in the final paper product or (b) directly to said material. The fusion protein may be used with or without the microcapsule or microparticle, but is used with the material itself when no microcapsule or microparticle is present. The compositions can be used to deliver materials that are beneficial in the processing of the paper or which are beneficial in the final paper product, including, for example, antibacterial agents, antifungal agents, perfume or fragrance, flavourants, surfactants, insect repellents, moisturising agents, organic solvents, binding agents for the paper and adhesives. Examples of uses in the paper industry include: wallcovering (e.g., wallpaper and border) manufacture and treatment, where the compositions of the invention may be used as cleaners, for aroma release, for colour reactivity, for stain removal or for coating; in the production of specialty paper products such as anti-odour shoe liners, moth repellents, tissues with additives or extra benefits, packaging paper having controlled release of fungicides, packaging that will disintegrate easily when required, food wrappers that have labels impregnated with food aroma; in the manufacture or printing of newspaper; to release molecules that are used to simplify the paper production process or allow strengthening e.g., strengthened paper for furniture production or kitchen roll; to produce scented books; for the restoration or preservation of books or manuscripts; in paper diagnostics (e.g., for fitting to urinals); in the application of an active material to toilet tissue; as a paper money cleaner or sanitiser; in the production of washable cards (such as identity cards or driving licences); impregnated wipes for cleaning spectacles or car windscreens; cloths for the targeted deposition of actives to kitchen surfaces; in the treatment of greaseproof paper to make it non-stick; in the formation of labels for packaging (such as cartons, bottles and cans, etc) that will disintegrate within the shelf life of the product; and in the production of paper comprising washing powder and/or fabric conditioner.

Other applications include the treatment of non-paper cellulosic articles. It can invention also be envisages a method of treating a cellulosic substrate with a fusion protein of the invention. Examples include treatments for wooden curtain poles e.g., for perfume release; dust repellents e.g., for spraying on lampshades; carpet fresheners; sprays for refreshing paper drawer liners; for the urine-triggered release of perfume or odour neutraliser; to form soft furniture binders; and the treatment of wooden items such as bread bins and chopping boards. Compositions may also be used in the veterinary field , for example as a treatment for pet litter or in the production of flea collars for pets.

Some applications of fusion proteins are described hereafter.

*a. Carbohydrate Anchor Therapeutic System*

As a first typical pharmaceutical application, a fusion protein is provided which comprises a cellulose binding domain (as a typical example of a carbohydrate binding domain) available for surface binding, and an anti-body domain recognising gelatine micro-capsules containing a therapeutic agent. The pharmaceutical application for this construct is useful, for example in dressings, wound care products and medical devices for internal or external use comprising cellulose or another carbohydrate. A suitable dressing is, for example, a cotton dressing containing a bound CBD construct where the micro-capsule contains an anti-microbial agent or antibiotic to be released through the action of microbial proteolytic enzymes. A medical device that benefits from this invention includes, for example, a urinary catheter designed for long term use without the risk of frequent urinary tract infections. A therapeutic advantage in both examples is the release of anti-microbial agents only in the event of significant infection thereby reducing the spread of anti-biotic resistance among normal bacterial populations. Examples of drugs include antibiotics of the penicillin type e.g. ampicillin, amoxycillin, aziocillin, benzylpenicillin, cloxacillin and also many cephalosporins. Other antibiotics include but are not restricted to doxycyline, tetracycline, cefaperazone, chloramphenicol and cefoxitin. The carbohydrate binding domain may be used in this approach to bind at a therapeutically useful binding site. For example a therapeutically useful binding site that may be recognised by the carbohydrate binding domain is the HIV envelope glycoprotein expressed on the surface of virus particles and also by infected cells and may therefore provide the means to more effectively target HIV drugs.

b. *Biphasic Delivery System*

A construct is provided containing a cellulose binding domain (as a typical example for a carbohydrate binding domain) bound to a micro-particle or micro-capsule comprising cellulose and at least one therapeutic agent together with an anti-body domain recognising gelatine micro-capsules containing at least one therapeutic agent. An advantage of this construct is the ready formation of a depot of at least one therapeutic agent for biphasic release. This is particularly important for the delivery of certain hormones such as insulin for the treatment of diabetes. Insulin is normally released in a biphasic manner from the pancreas following the ingestion of food, an initial rapid release of insulin is followed by sustained slow release of insulin. This insulin release profile is important in maintaining normal glucose homeostasis but is difficult to achieve using conventional formulation techniques. In the present invention the biphasic release of insulin is controlled by the composition of the two separate insulin-containing micro-capsules or bound to either the carbohydrate binding domain or an anti-body binding domain recognising gelatine micro-capsules formulated for rapid or sustained release. Insulin-containing fusion proteins of this type are suitable either for depot injection, oral delivery (providing they are protected from the normal digestive processes) or by inhalation enabling biphasic delivery of insulin to be achieved from the lungs. An advantage of insulin delivery via the oral or pulmonary route is the elimination or reduced need for daily insulin injections or perhaps the more effective treatment of type 2 diabetics normally treated with oral hypoglycaemic agents.

c. *Site Specific Delivery System*

A fusion protein suitable for site-specific delivery uses the cellulose binding domain (as typical example of a carbohydrate binding domain) to bind a carbohydrate-containing micro-capsule or micro-particulate containing at least one therapeutic agent, whereas another portion of the fusion protein comprises the site-specific targeting moiety. This targeting moiety is preferably in the form of an anti-body recognising a specific disease site or alternatively a receptor expressed by cells of tissue at or near the site where the therapeutic agent is most required. An example of a suitable application is the delivery of an anti-cancer drug to a tumour site. More specifically, this may involve the use of a fusion protein with an anti-body region recognising prostate specific antigen expressed by prostate tumour cells and a micro-particle or micro-capsule bound to the carbohydrate binding domain containing anti-cancer drug that would therefore be concentrated at the disease site. This will have the effect of both increasing therapeutic efficacy by forming a higher drug concentration at the tumour site while reducing the overall dose for the same therapeutic effect, thereby reducing the likelihood and severity of side effects. Examples of anti-cancer drugs include alkylating agents, chlorabucil, melphalan, busalfan, lomustine, carmustine, estramustine and thiotepa; cytotoxic antibiotics, doxorubicin, epirubicin, daunorubicin, mitoxantrone, bleomycin and mytomycin; anti-metabolites, methotrexate, cytarabine, fludarabine, cladribine, gemictabine, fluorouracil; vinca alkaloids, vinblastine, vincristine, vindesine, vinorelbine, together with other anti-neoplastic agents such as amsacrine, crisantapase, dacarbazine, temozolomide, pentostatin, carboplatin, cisplatin, oxaliplatin, paclitaxel and docetaxel.

d. *Site Specific Biphasic Delivery System (Multi-binder system)*

The fusion protein which is provided here combines the advantages of both site-specfic and biphasic delivery using an anti-body construct that has at least three binding sites one of which is a carbohydrate binding site. The carbohydrate binding site is used to bind, e.g., a micro-particle or micro-capsule containing at least one therapeutic agent with the release rate being determined by the composition of the micro-capsule or micro-particle. In addition, at least one binding domain is used to bind an additional micro-capsule containing at least one therapeutic agent with the release rate being determined by the composition of the micro-capsule. An example of a therapeutic application for the site specific biphasic delivery system is the enhanced treatment of Parkinson's disease. Parkinson's disease which is characterised by a progressive loss of motor control is treated with the drug levodopa, a precursor for the neuro-transmitter dopamine in the brain. Unfortunately, only small amounts of levodopa are absorbed across the blood-brain barrier and as a result levodopa is available for conversion into dopamine by peripheral tissues causing cardiovascular side-effects, nausea and vomiting. To reduce the conversion of levodopa by peripheral tissues a dopa-carboxylase inhibitor is used such as carbidopa. The present invention is contemplated to improve the treatment of Parkinson's disease in the following way: The fusion protein would contain an antibody recognising a binding site expressed on the blood brain barrier such p-glycoprotein. Once bound to the blood-brain barrier a bolus release of dopa-carboxylase inhibitor would be released from one of the bound micro-capsules to reduce peripheral dopamine production while a sustained release of levodopa would occur from another bound micro-capsule. The close proximity to the blood-brain barrier of levodopa release would facilitate absorption into the brain with the effect of either increasing therapeutic efficacy or reducing the dose required for controlling symptoms.

An alternative application of the site-specific biphasic delivery system is to enhance cancer chemotherapy as described in a previous example above. Instead of just relying on one bound micro-capsule or micro-particle containing at least one therapeutic agent two or more could be directed to the tumour site. For example, a fusion protein comprising a carbohydrate binding domain bound to a carbohydrate-containing micro-capsule containing at least one anti-cancer agent, another binding domain bound to a gelatine micro-capsule containing at least one alternative anti-cancer agent and a binding domain recognising the tumour site or tissue close to the tumour site. A particular advantage of this arrangement is the ability to combine the site-specific delivery of at least two different anti-tumour agents of widely different physical chemistry. For example, the carbohydrate containing micro-capsule or micro-particle may contain at least one relatively water soluble therapeutic agent whereas the gelatine micro-capsule may be used to contain at least one oil soluble therapeutic agent. A particular advantage of this combined site specific delivery is overcoming drug resistance of certain tumours resulting from prior chemotherapy.

The invention will now be described with references to the following examples which are intended to illustrate the invention and not to be in any way limiting on the scope of the invention. In the examples and throughout this specification all percentages are percentages by weight unless indicated otherwise.

Unless otherwise stated all materials were obtained from Sigma Chemical Company, UK).

## Example 1

**Deposition of a molecule to cotton wool, tissue and string surfaces.**

In this example, a CBD-llama antibody fusion protein was used. The construction and production of such CBD fusion proteins has been described (WO0146757, WO0146356 and WO0146364) and was carried out in an analogous manner in this example. In this instance, the antibody domain recognises a red sulphonated dye, Reactive Red six (RR6 - supplied from ICI Chemical Co.). This dye can then be attached to a molecule of interest via its reactive triazine group. For this example, RR6 was coupled to Bovine Serum Albumin (BSA) as follows:

*Preparation of BSA-RR6:*

10mg RR6 dissolved in 10ml borate buffer (0.1M $Na_2B_4O_7.10H_2O$ + 0.05M NaCl, pH9.5). Separately, 1g BSA was also dissolved in 10ml borate buffer. The BSA and RR6 solutions were combined and mixed on a rotary shaker overnight at room temperature. After 24 hours, the resulting solution was diluted two fold with 0.5M Tris-HCl to block any unreacted dye sites. This was mixed for 2 hours. The resulting mixture was then added to a Centricon 30s (fitted with a 30kDa membrane - Supplied from Millipore, UK) and spun repeatedly until the filtrate component was clear. This therefore separated any unbound RR6 from the BSA. Phosphate Buffered Saline (PBS) was then added to the upper reservoir, containing the BSA-RR6 concentrate. The upper reservoir was inverted and spun to collect the desired BSA-RR6 solution.

*Using the CBD fusion to deposit BSA RR6 to cotton wool, tissue and string:*

The CBD fusion (100μg/ml) and 20μl BSA-RR6 were added together and incubated in a total volume of 100μl water for 30 minutes, 25°C with shaking at 150 rpm. As a negative control, no CBD fusion was added to the BSA-RR6 and water. Following this premixing, 900μl water was added and at this point the cellulosic material was added. All cellulosics were commercial grade materials (cotton wool purchased from Sainsbury's Plc, UK, Tissue purchased from Kleenex, UK). These samples were incubated for 30 minutes, 25°C with shaking at 150 rpm. Following this binding step, the cellulosic samples were rinsed by rinsing the materials in 2 x 100ml beakers of water. The cellulosic samples were then air dried. The materials were then scanned to illustrate the binding and deposition of the red dye conjugate.

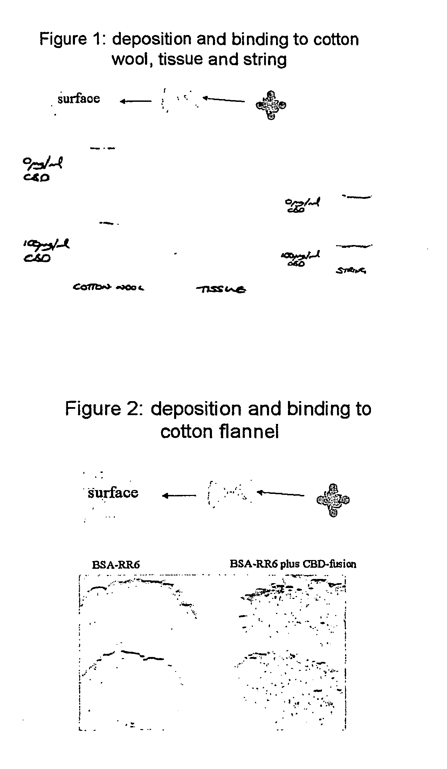

The results are shown in Figure 1. This clearly shows the ability of the CBD fusion to deposit the target molecule to a cellulosic material with application in the pharmaceutical and cosmetics field. In the absence of the fusion protein, the dye is unable to bind to the cellulose.

## Example 2

**Deposition of a molecule to cotton flannel.**

The use of terry towelling is extensive in the medical environment. Using the same materials as described in Example 1, the CBDanti RR6 fusion protein was used to deposit RR6 to this cellulosic material.

*Using the CBD fusion to deposit BSA RR6 to terry towel:*

The experimental procedure was the same as that described in Example 1 except that the cellulosic surface was replaced with a 2cm$^2$ piece of terry towelling (Brennard Textiles, UK). The results are shown in Figure 2. Clearly, the presence of the fusion dramatically improves the delivery of the RR6-BSA conjugate to the cellulosic surface.

## Example 3.

### Deposition of a molecule to cellulosic paper structures.

Cellulosic papers are one of the simplest commercial forms and the most abundant in product form. In this example, the CBD fusion protein was used to deliver RR6-BSA to cellulose based tea bag materials.

*Using the CBD fusion to deposit BSA RR6 to paper cellulosics:*

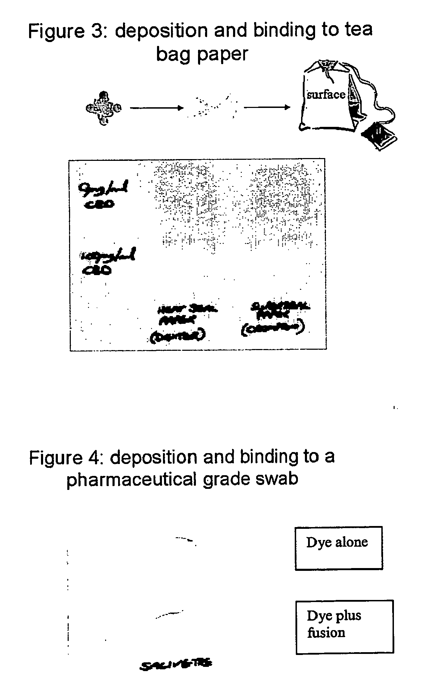

The experimental procedure was the same as that described in Example 1 except that the cellulosic surface was replaced with a piece of either Heatseal™ paper (Dexter) cut to 1.5 x 1.5cm or Superseal™ paper (Crompton) cut to 1.5 x 1.5cm. These are commercial grade paper products kindly supplied by Lipton UK. The results are shown in Figure 3. The presence of the fusion protein improves the delivery of the RR6-BSA conjugate to the cellulosic surface.

## Example 4.

### Deposition of a molecule to cotton buds and to a salivette.

This example was generated to demonstrate the ability to deposit benefit agents onto cellulosic materials used in the pharmaceutical and cosmetics field.
Cotton buds were purchased from Sainsbury's Plc. A neutral cotton wool salivette swab was obtained from Sarstedt, Germany.
RR6 CBD fusion protein was used at 0 and 100μg/ml. BSA-RR6 was used as an example of a desired active to be deposited onto the salivette. The CBD fusion and 20μl BSA-RR6 were incubated in a total volume of 100μl water for 30 minutes at 25°C with shaking at 150rpm. As a negative control, no CBD fusion was added to the BSA-RR6 and water. 900μl water was added plus the salivette and they were incubated for 30 minutes at 25°C with shaking at 150rpm. Samples were rinsed by placing in 2 x 100ml beakers of water for 10 seconds each and by then placing in 5ml water and washing for 30 minutes at 25°C with shaking at 150rpm. Samples were allowed to air dry. The resulting salivettes were then scanned to demonstrate the deposition capability. The results are shown in Figure 4.

*Cotton buds:*

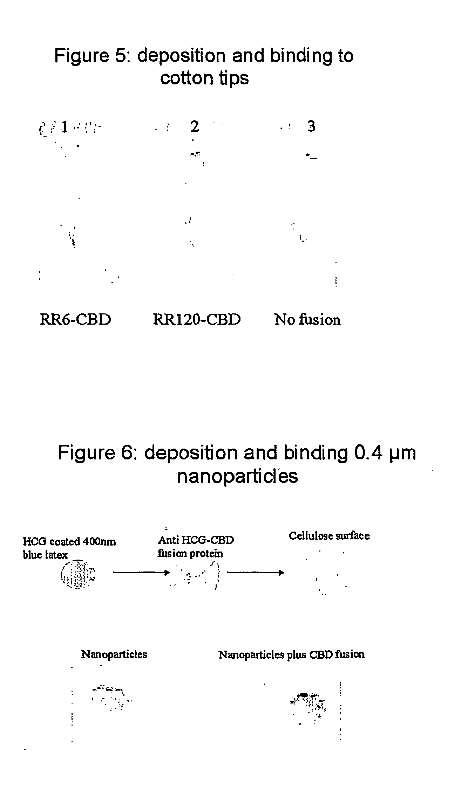

This experiment was constructed as a model system to show that cotton buds/swabs could be pre-loaded with any active using the CBD-fusion protein. In this instance, the inclusion of a CBD anti RR120 (Reactive Red 120) fusion was used to demonstrate the specific deposition capability of the protein. Therefore the CBD-RR120 should not improve the deposition of the RR6-BSA and acts as another negative control. The cotton buds were cut in half across the width of the shaft to produce single ended buds. Solutions of each of the fusion proteins were made up into PBST to give a concentration of 100μgml, 1ml of each was dispensed into eppendorf tubes and 1ml of PBST was dispensed into a third eppendorf tube as a control. Swabs were pre-wetted with PBST (Phosphate buffered saline including Tween) and the excess blotted off onto clean tissue. One swab was placed into each of the solutions for 30 minutes incubation at room temperature (20°C ±1°C). The swabs were then washed in three changes of PBS to remove any unbound material and the excess liquid was gently blotted away. A 1/50 dilution of BSA-RR6 conjugate was prepared in PBSA and 1ml was dispensed into each of 3 eppendorf tubes. A cotton bud was placed into each and incubation was carried out for a further 30 minutes at room temperature. The cotton buds were removed and given two washes in PBS and a final wash in distilled water. Excess liquid was blotted away and the buds were allowed to dry at 60°C. The results are shown in Figure 5. Clearly, the deposition of the RR6-BSA is only achieved in the presence of the CBD-anti RR6 fusion protein.

## Example 5

**Deposition of particles onto cellulose.**

A key attribute of the invention in one embodiment is the delivery of relatively large amounts benefit agents to surfaces from particles. Importantly, the bigger the particle the greater the volume, and therefore it is important to show effective binding across the envisaged range of particles. Using the particle as the antigen it is possible to encapsulate many benefit agents into the capsule with the antibody domain generated to recognise the outside of the capsule.

When targeting cellulosic surfaces, some particles will become physically entrapped into the structure. The following examples show that a range of particle sizes can improve the deposition of particles over that retained solely by physical interactions.

Average particle sizes were determined using an Olympus BX41™ microscope fitted with an Olympus DP50™ image capture unit and a 10 micron ($\mu$m) increment graticule slide obtained from Pyser, UK.

*Using the CBD fusion protein to deposit 0.34pm particles to cellulose:*

A CBD-anti Human Choronic Gonadotrophin (HCG) ScFv fusion was used to demonstrate the delivery of HCG coated latex ranging from 0.34 to 80$\mu$m diameter to cellulose as follows:

*Preparation of HCG latex beads:*

A 300$\mu$l sample of 1% by weight latex solids was diluted into 3ml 10mM borate (containing 0.01% by weight Thimerosal, pH8.5). This was then centrifuged for 10 minutes at 13000rpm. Supernatant was then removed and the pellet was added to 0.0042 mg/ml HCG. The sample was then vortexed and sonicated for 10 seconds. The sample was then left to incubate for 2 hours at 32°C with an end over end mixer. Following incubation, 50$\mu$l of a 200mg/ml BSA solution was then added. Samples were then further mixed in an end over end mixer for 90 minutes. Samples were then centrifuged for 10 minutes at 13000rpm, the supernatant removed, and the pellet resupended in borate buffer. Particles were then stored at 4°C until use. ELISA confirmed the presence of HCG on the surface of the beads.

*Deposition experiments:*

Blue latex was used for 340nm diameter experiments, whereas fluorescent green latex was used for 10$\mu$mand 31$\mu$m bead experiments.

340nm beads: hole punched pieces of terry towelling were used as surfaces for depositing particles. Blue latex particles sensitised with HCG were used. 5$\mu$l HCG latex was added to 15 $\mu$l ScFv3299CBD (lmg/ml) and mixed for 15 minutes. This was then added to pre-wetted cotton disk in 1 ml PBST. Samples were then incubated for 30 minutes at room temperature on an end over end mixer. Following incubation, the cotton disks were removed and washed in 10 ml PBST. Finally, the cotton disks were air dried and mounted onto white card. The negative control was particles without fusion protein. The cotton disks were scanned and the results are shown in Figure 6.

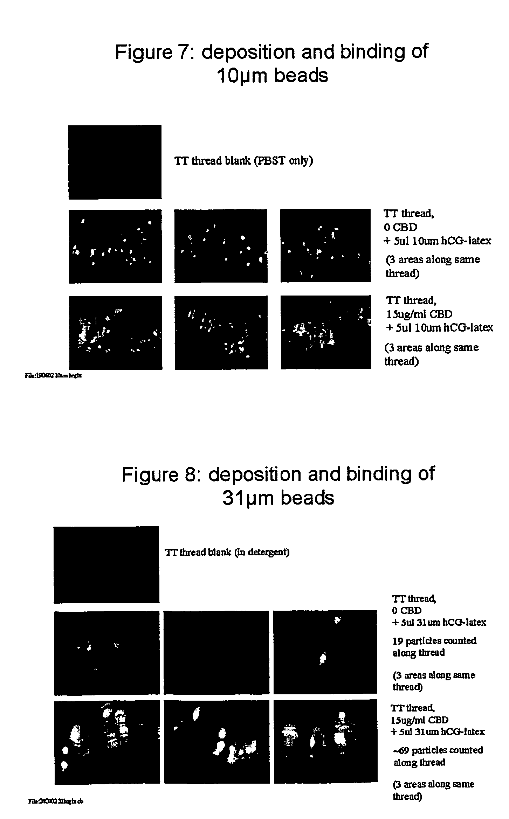

10$\mu$m beads: As a cellulosic surface, terry towelling threads were used (25.4 mm long). The fusion protein used was ScFv3299CBD at 15$\mu$g/ml or omitted in the blank control. The CBD fusion and terry towelling threads were incubated in a total volume of 1ml PBST, for 30 minutes, at room temperature with shaking. As a negative control, threads were incubated in 1ml PBST. Threads were washed in 1ml PBST for 30 minutes, at room temperature with shaking. Threads were placed in a solution containing 5$\mu$l HCG-latex (10$\mu$m) plus 995$\mu$l PBST for 30 minutes, at room temperature with shaking. Threads were washed in 1ml PBST for 30 minutes, at room temperature with shaking and were then observed under a microscope using fluorescence to visualise bound particles. These are shown in Figure 7.

31$\mu$m beads: The CBD fusion protein and terry towelling threads were incubated in a total volume of 1ml PBST, for 30 minutes, at 25°C with shaking at 130rpm. As a negative control, threads were incubated in 1ml PBST. Threads were washed in 1ml PBST for 30 minutes, at 25°C with shaking at 130rpm. Threads were placed in a solution containing 5$\mu$l HCG-latex (31$\mu$m) plus 995$\mu$l PBST for 30 minutes, at 25°C with shaking at 130rpm. Threads were washed in 1ml PBST for 30 minutes, at 25°C with shaking at 130rpm and were then observed under a microscope using fluorescence. This data is shown in Figure 8.

Notably in each case the CBD fusion improves the deposition of particles over the particle size range. The data clearly demonstrates that it is possible to target a range of particle sizes to surfaces. In addition, the deposition allows a significant improvement in deposition over physical entrapment especially when the particle size increases. This finding, coupled with increasing payload volumes with particle size, indicates the benefit of this deposition technology.

### Example 6

**Deposition of particles to plant material.**

This example demonstrates the delivery of gelatine particles to plant material using the CBD-anti fusion protein described in Example 1. The particles were made by the standard coacervation methods.

*Materials*

Fusion proteins: CBD-antiRR6, CBD-anti RR6-CBD, Anti RR6$_2$-CBD RR6 dyed coacervate particles (containing Nile red)

Freshly cut grass

PBST

Each fusion protein type was made up at concentrations of 300μg/ml in PBST and 100μl of < 60μm particles were added. The tubes were placed onto a roto-torque and left turning slowly at room temperature for 30 minutes. Blades of grass were washed in water and then PBST and gently blotted. Small pieces of approximately 15mm were placed into the tubes containing the particles and the fusion protein and incubation was continued for 30 minutes. As a control, grass was placed into a tube containing particles only for 30 minutes. Blades of grass were removed from the eppendorf tubes and washed in two changes of PBS. Fluorescence microscopy was used to examine the blades of grass for bound particles.

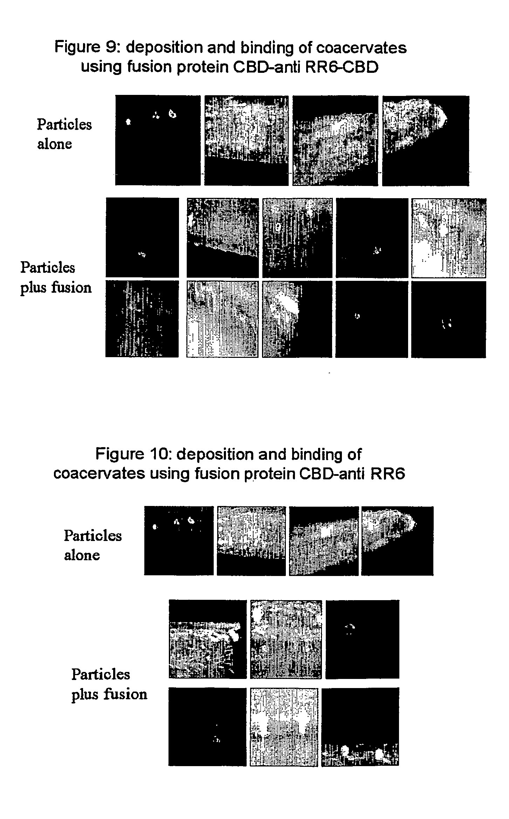

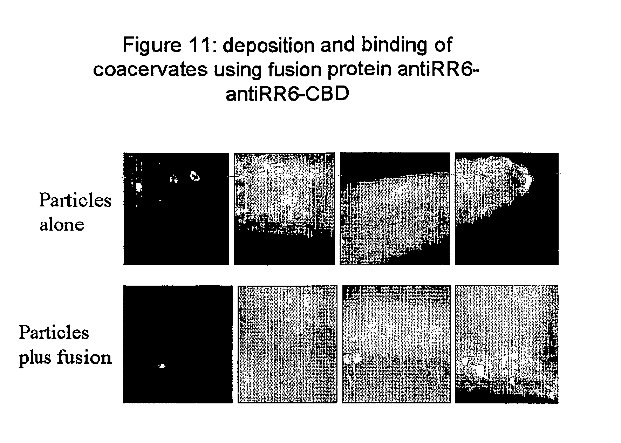

The data is shown in Figures 9, 10 and 11.

Using these different fusion protein constructs, it was surprisingly found that deposition of these particles was enhanced in the presence of the extra CBD.

The potential of encapsulating agrochemical benefit agents such as pesticides into capsules is very straightforward. The following demonstrates the ability to encapsulate deltamethrin into capsules:

Gelatin and Gum arabic were made up as 3% by weight solutions in water, to fully dissolve they were stirred for 1 hour and heated to 60°C.

Fifty milligrams of deltamethrin was weighed out and added to 0.4ml of ethyl alcohol, this was mixed and added to 3mls of sunflower oil (Deltamethrin was obtained from QMX Laboratories Ltd, UK). This mixture was vortexed briefly to mix before being added to 50ml of gum arabic. The Ultraturax (IKA-Werke, Germany) was used at moderate speed for 40 seconds to emulsify the ingredients, finally 50mls of gelatin was added. This mixture was then stirred with a stirrer bar and base and glucono-delta lactone was added dropwise to adjust the pH to 3.8 (this would allow the coacervation process to take place). The stirrer bar was removed and stirring was continued at a moderate speed with an overhead stirrer. After 1 hour, the stirring was stopped and the coacervate particles were washed over a 60μm sieve and placed into PBS.

The particles were hardened by adding 0.1% by weight glutaraldehyde (working concentration) and placing them in a falcon tube on a roller bed for two hours at room temperature 20°C±1°C. The particles were washed and stored in PBSA at 4°C. Samples were viewed under the light microscope and were confirmed as containing deltamethrin by GC-MS analysis of an extract from the particles using ethyl acetate as the extraction solvent.

### Example 7

**Targeting Gelatin coacervates to paper with VHH-CBD Fusion proteins.**

*Materials*

Dexter paper (2039) cut into discs with 6mm diameter. Gelatin/Gum arabic coacervates filled with Nile red and dyed on the outside with RR6 dye.

Fusion proteins: VHH-CBD; VHH-VHH-CBD; VHH-CBD-CBD. All VHH (antibody-derived parts of the fusion protein) specific for RR6 dye, as in Example 1, produced as described above.

Phosphate buffered saline (PBS)

Eppendorf tubes

Sterilin microtitre plates

*Method*

1) The fusion proteins were added to eppendorf tubes at the following concentrations: 25, 50 and 100μg/ml. To this a 25μl mixture of coacervate particles in PBS was added and the sealed tubes were incubated with gentle shaking for 20 minutes. PBS was then added to bring each tube volume up to 0.5ml, the final concentrations were therefore 50, 100 and 200μg/ml of fusion protein. A control of particles only was prepared by adding 25μl of particles to 475μl of PBS.
2) 22 discs of paper were placed into the wells of a microtitre plate and 100μl of PBS was dispensed to each well. The paper was allowed to soak for 5 minutes before pipetting out the PBS and discarding it. 100μl duplicates of the fusion and particle dilutions were dispensed and also duplicates of the particles only were dispensed. The samples were left to incubate at room temperature 21°C ± 1°C for 60 minutes.
3) The samples were washed in beakers containing 100ml PBSA, each duplicate pair was placed into a beaker and stirred gently for 20 seconds, the discs were then allowed to settle before being removed with forceps and being placed into fresh wells prior to analysis.
4) Each disc was methodically viewed under fluorescence and particles were counted and recorded for each disc. The counts are recorded in Table 1.

Table 1. Particle counts on paper discs treated with CBD-VHH fusion proteins and particles or particles only

| TRIAL | Fusion concentrat ion | Particle counts on each duplicate | Average particle count |
|---|---|---|---|
| Particles only | N/A | 9<br>8 | 9 |
| VHH-CBD | 50μg/ml | 37<br>44 | 41 |
| | 100μg/ml | 21<br>25 | 23 |
| | 200μg/ml | 22<br>25 | 24 |
| VHH-VHH-CBD | 50μg/ml | 87<br>67 | 77 |
| | 100μg/ml | 45<br>32 | 39 |
| | 200μg/m | 49<br>50 | 50 |
| VHH-CBD-CBD | 50μg/ml | 107<br>112 | 110 |
| | 100μg/ml | 31<br>118 | 75 |
| | 200μg/m | 69<br>41 | 55 |

All of the trial samples containing fusion protein demonstrated more particles on the paper discs. The overall trend indicated that the lower concentration of fusion protein added to the particles was more effective at delivering particles to the surface.
These data suggest that targeting to a paper surface is more effective if the construct has two CBD domains.

**Claims**

1. A method of delivering an agrochemical benefit agent to a site in or on a plant, which comprises treating the plant with an agrochemical composition or kit of parts comprising (1) a micro-particle or micro-capsule comprising at least one agrochemical benefit agent and (2) a fusion protein which comprises a first binding domain which is a carbohydrate binding domain and a second binding domain capable of binding to (a) a ligand or specific site which forms part of a plant, or (b) said microcapsule or micro-particle, wherein the first or second binding domain is capable of binding to said microcapsule or microparticle.

2. A method as claimed in claim 1 wherein the benefit agent is selected from pesticide, herbicides, plant growth stimulating agents, insect attractants or repellents and crop protecting agents.

3. A method as claimed in any one of claims 1 to 2, wherein the carbohydrate binding domain is a cellulose binding domain.

4. A method as claimed in any one of claims 1 to 3, wherein the second binding domain is an antibody or fragment thereof.

5. A method as claimed in claim 4, wherein the antibody or fragment thereof is a Heavy Chain antibody obtainable from the group of Camelidae.

6. A method as claimed in any one of claims 1 to 5, wherein the fusion protein further comprises a third binding domain which is capable of binding to said micro-particle or micro-capsule, or to a desired antigen.

7. A method as claimed in any one of claims 1 to 6, wherein the carbohydrate binding domain comprises a cellulose binding domain obtainable from a fungal enzyme origin selected from the group consisting of *Humicola*, *Trichoderma*, *Thermomonospora*, *Phanerochaete*, *Aspergillus* or from a bacterial enzyme origin selected from the group consisting of *Bacillus*, *Clostridium*, *Streptomyces*, *Cellulomonas* and *Pseudomonas*.

8. A method as claimed in any one of claims 1 to 7, wherein the first binding domain is connected to the second binding domain by means of a linker peptide group comprising at least 2 to 15 amino acid residues, preferably 2 to 5 amino acid residues.

**Patentansprüche**

1. Verfahren zum Abgeben eines agrochemischen Mittels mit günstigen Eigenschaften an eine Stelle oder auf eine Pflanze, welches Behandeln der Pflanze mit einer agrochemischen Zusammensetzung oder einem agrochemischen Kit aus Teilen, umfassend (1) ein Mikropartikel oder eine Mikrokapsel, das/die wenigstens ein agrochemisches Mittel mit günstigen Eigenschaften umfasst, und (2) ein Fusionsprotein, umfassend eine erste Bindungsdomäne, die eine Kohlenhydratbindungsdomäne ist, und eine zweite Bindungsdomäne, die zur Bindung an (a) einen Liganden oder eine spezifische Stelle, welche(r) einen Teil einer lebenden Pflanze bildet, oder (b) die Mikrokapsel oder das Mikropartikel fähig ist, wobei die erste oder zweite Bindungsdomäne zur Bindung an den Mikrokapseln oder das Mikropartikel fähig ist, umfasst.

2. Verfahren, wie es in Anspruch 1 beansprucht ist, wobei das Mittel mit günstigen Eigenschaften aus Pestiziden, Herbiziden, das Pflanzenwachstum stimulierenden Mitteln, Insektenlockstoffen oder Insektenabwehrmitteln und Pflanzenschutzmitteln ausgewählt ist.

3. Verfahren, wie es in einem der Ansprüche 1 bis 2 beansprucht ist, wobei die Kohlenhydratbindungsdomäne eine Cellulosebindungsdomäne ist.

4. Verfahren, wie es in einem der Ansprüche 1 bis 3 beansprucht ist, wobei die zweite Bindungsdomäne ein Antikörper oder ein Fragment davon ist.

5. Verfahren, wie es in Anspruch 4 beansprucht ist, wobei der Antikörper oder das Fragment davon ein Schwerketten-Antikörper ist, der aus der Gruppe von Camelidae erhältlich ist.

6. Verfahren, wie es in einem der Ansprüche 1 bis 5 beansprucht ist, wobei das Fusionsprotein außerdem eine dritte Bindungsdomäne umfasst, die zur Bindung an das Mikropartikel oder die Mikrokapsel oder an ein gewünschtes Antigen fähig ist.

7. Verfahren, wie es in einem der Ansprüche 1 bis 6 beansprucht ist, wobei die Kohlenhydratbindungsdomäne eine Cellulosebindungsdomäne umfasst, die von einem fungalen Enzym mit Herkunft, ausgewählt aus der Gruppe, bestehend aus *Humicola, Trichoderma, Thermomonospora, Phanerochaete, Aspergillus*, oder von einem bakteriellen Enzym mit Herkunft, ausgewählt aus der Gruppe, bestehend aus *Bacillus, Clostridium, Streptomyces, Cellulomonas* und *Pseudomonas*, erhältlich ist.

8. Verfahren, wie es in einem der Ansprüche 1 bis 7 beansprucht ist, wobei die erste Bindungsdomäne mit Hilfe einer Linkerpeptidgruppe, die wenigstens 2 bis 15 Aminosäurereste, vorzugsweise 2 bis 5 Aminosäurereste, umfasst, gebunden wird.

## Revendications

1. Procédé pour délivrer un agent agrochimique bénéfique à un site dans ou sur une plante, qui comprend le traitement de la plante avec une composition ou un kit de pièces agrochimique comprenant (1) une microparticule ou microcapsule comprenant au moins un agent agrochimique bénéfique et (2) une protéine de fusion qui comprend un premier domaine de liaison qui est un domaine de liaison à un hydrate de carbone et un deuxième domaine de liaison capable de se lier à (a) un ligand ou un site spécifique qui forme une partie d'une plante, ou (b) ladite microcapsule ou microparticule, dans lequel le premier ou deuxième domaine de liaison est capable de se lier à ladite microcapsule ou microparticule.

2. Procédé selon la revendication 1, dans lequel l'agent bénéfique est choisi parmi les pesticides, les herbicides, les agents stimulant la croissance des plantes, les insecticides appâts ou les insectifuges, et les agents de protection des cultures.

3. Procédé selon l'une quelconque des revendications 1 et 2, dans lequel l'agent de liaison à un hydrate de carbone est un agent de liaison à une cellulose.

4. Procédé selon l'une quelconque des revendications 1 à 3, dans lequel le deuxième domaine de liaison est un anticorps ou un fragment de celui-ci.

5. Procédé selon la revendication 4, dans lequel l'anticorps ou le fragment de celui-ci est un anticorps de chaîne lourde pouvant être obtenu à partir du groupe des camélidés.

6. Procédé selon l'une quelconque des revendications 1 à 5, dans lequel la protéine de fusion comprend en outre un troisième domaine de liaison qui est capable de se lier à ladite microparticule ou microcapsule, ou à un antigène souhaité.

7. Procédé selon l'une quelconque des revendications 1 à 6, dans lequel le domaine de liaison à un hydrate de carbone comprend un domaine de liaison à une cellulose pouvant être obtenu à partir d'une enzyme d'origine fongique choisie dans le groupe constitué par Humicola, *Trichoderma, Thermomonospora, Phanerochaete, Aspergillus* ou à partir d'une enzyme d'origine bactérienne choisie dans le groupe constitué par *Bacillus, Clostridium, Streptomyces, Cellulomonas* et *Pseudomonas*.

8. Procédé selon l'une quelconque des revendications 1 à 7, dans lequel le premier domaine de liaison est connecté au deuxième domaine de liaison au moyen d'un groupe peptidique lieur comprenant d'au moins 2 à 15 résidus d'acides aminés, de préférence de 2 à 5 résidus d'acides aminés.

## Figure 1: deposition and binding to cotton wool, tissue and string

## Figure 2: deposition and binding to cotton flannel

## Figure 3: deposition and binding to tea bag paper

## Figure 4: deposition and binding to a pharmaceutical grade swab

| Dye alone |
| Dye plus fusion |

## Figure 5: deposition and binding to cotton tips

1         2         3

RR6-CBD     RR120-CBD     No fusion

## Figure 6: deposition and binding 0.4 μm nanoparticles

HCG coated 400nm blue latex     Anti HCG-CBD fusion protein     Cellulose surface

Nanoparticles     Nanoparticles plus CBD fusion

## Figure 7: deposition and binding of 10μm beads

TT thread blank (PBST only)

TT thread,
0 CBD
+ 5ul 10um hCG-latex

(3 areas along same thread)

TT thread,
15ug/ml CBD
+ 5ul 10um hCG-latex

(3 areas along same thread)

File:190402 10um beads

## Figure 8: deposition and binding of 31μm beads

TT thread blank (in detergent)

TT thread,
0 CBD
+ 5ul 31um hCG-latex

19 particles counted along thread

(3 areas along same thread)

TT thread,
15ug/ml CBD
+ 5ul 31um hCG-latex

~69 particles counted along thread

(3 areas along same thread)

File:240402 31um beads cb

Figure 9: deposition and binding of coacervates
using fusion protein CBD-anti RR6-CBD

Particles
alone

Particles
plus fusion

Figure 10: deposition and binding of
coacervates using fusion protein CBD-anti RR6

Particles
alone

Particles
plus fusion

Figure 11: deposition and binding of coacervates using fusion protein antiRR6-antiRR6-CBD

Particles alone

Particles plus fusion

Figure 12

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5738984 A
- US 6124117 A
- WO 9424158 A
- WO 0146357 A
- WO 9800500 A
- US 6331416 B
- WO 9404678 A

- WO 9425591 A
- WO 9429457 A
- EP 0194276 A
- WO 0146757 A
- WO 0146356 A
- WO 0146364 A

**Non-patent literature cited in the description**

- **BENDAS G.** *Biodrugs,* 2001, vol. 15 (4), 215-224
- **BARNWELL SG et al.** *International Journal of Pharmaceutics,* 1996, vol. 128, 145-154
- **HENRISSAT B. ; DAVIES G.J.** *Plant Physiology,* 2000, vol. 124 (4), 1515-1519
- Cellulose Binding Domains: Classification and Properties. **PETER TOMME et al.** Enzymatic Degradation of Insoluble Carbohydrates. 1996

- *J. Mol. Biol.,* 1996, vol. 259, 957-969
- *Protein. Eng.,* 1996, vol. 9, 531-537
- *Bio/Technology,* 1995, vol. 13, 475-479
- **HOLLIGER et al.** *PNAS,* 1993, vol. 90, 6444-6448
- **GANI et al.** *J. Steroid Biochem. Molec. Biol.,* 1994, vol. 48, 277-282